# EUROPEAN PATENT APPLICATION

(11) **EP 1 674 064 A1**
(43) Date of publication of application: **28.06.2006**
(21) Application number: 05078021.2
(22) Date of filing: 22.12.2005
(51) Int. Cl.: A61F 13/15, A61L 15/20

(54) **Absorbent bed pad**

(30) Priority: 27.12.2004 US 20210
(71) Applicant: Horowitz, David, D.D.O. Quebec H9A 2C5 (CA)
(72) Inventor: Horowitz, David, D.D.O. Quebec H9A 2C5 (CA)
(74) Representative: Lucas, Brian Ronald

(57) **Abstract**

The present invention provides for an absorbent pad (10) for absorbing liquids exuded from a body, comprising a planar body (60) having a top surface (70), a bottom surface (80) and an outer perimeter (90) defining a middle portion; a particulate blended and dispersed within at least one of the top surface, the bottom surface or the middle portion of the planar body; the particulate has greater absorbent characteristics than the planar body, has a maximum dimension of 1/4" (6.4mm) and is peat moss.

## Description

### FIELD OF THE INVENTION

The present invention relates to absorbent materials and more particularly to an absorbent body for maintaining moisture away from a body when the pad is in use.

### BACKGROUND OF THE INVENTION

Absorbent pads are generally used to minimize exposure of the skin to moisture. Moisture alone can predispose the skin to serious injury and subsequent pressure ulcers, particularly to certain high-risk groups which include the elderly, individuals with spinal cord injuries or any individual with an impaired ability to reposition. Absorbent sheets or pads are often used to protect the skin of patients who are incontinent but can also include exposure to a variety of substances such as urine, stool, perspiration, or wound drainage.

Pads are absorbent products that are placed on top of, for example, a bed or chair and typically consist of three or more layers. The major structural components of the pad include a top layer, an absorbent core and a backsheet.

The top layer contains and conceals the underlying absorbent core while also being in direct contact with the skin. The top cover is generally a non-woven fabric, light weight and low density with a smooth soft web which permits fluid to flow freely into the underlying absorbent core but prohibits fluid from flowing out of the absorbent core -- regardless of the insult rate of flow. However, the top layer is not limited to non-woven fabrics and are also known to be formed from woven or knit fabrics.

To achieve high comfort, aesthetics and discretion, the absorbent core needs to be thin, soft and pliable. Conventional materials are based on a granular superabsorbent polymer (SAP) added to a cellulose fluff which typically absorbs about 5-7 times their own weight. The vast majority of superabsorbent materials are cross-linked polyacrylates that are partially neutralized. SAPs can soak up between 25-50 times their own weight in laboratory tests. Modern polyacrylates resemble granulated sugar in a dry state but tend to be difficult to incorporate into a layer and are also typically expensive and not as efficient as the traditional polyacrylates.

There are currently no alternatives to polyacrylates being used on a large scale today. Other absorbent materials have been tested in a few products, such as moss in sanitary towels, which has been found to result in an improved biodegradability but a generally lower absorption capacity. However, raw materials which are modified to improve their absorption capacity often result in reduced biodegradability. Traditional materials based on a mixture of fluff and SAP powder are soft but rather bulky, uncomfortable and lack any integrity. Bonded non-woven products offer good integrity but are often relatively stiff because of excessive inter fibre bonding. Thickness can be reduced by web densification, however, thinness means less void volume and porosity, hence poor absorbency.

The third layer, or back sheet, may be a polyethylene film structure or a film/non-woven composite, for example the so called "textile back sheet" (TBS), which can also be breathable. The back sheet prohibits moisture from contacting the object on which the pad is positioned while in use.

Absorbent articles having a combination of materials are known in the art. For example, U.S. 5,681,300 is directed to an absorbent article for use as a diaper, sanitary napkin or an adult incontinence device having a blended absorbent core. The core is described as being a homogenous blend of at least two groups of fibres. The first group of fibres can be any synthetic fibre, such as polypropylene, polyethylene, rayon etc. which are inherently hydrophilic or rendered hydrophilic by treatment. The second group of fibres are described as having particles greater than 1/4" (6.4mm) in size and having any material which is suitable for the acquisition layer. The core is further described as possibly including "additional materials", one such additional material being identified as peat moss. However, there are no teachings with respect to the blending or blending techniques used to include the peat moss in the absorbent core.

U.S. 6,146,725 provides for an absorbent composition which generates an antiseptic when exposed to water for killing organisms in the fluid. A dye marker is used to visually indicate leakage has occurred for the safety of a transport team. While U.S. '725 contemplates the use of cellulose fibres with peat moss, the composition is directed to hygienic improvements and not improved absorbencies. Further, the reference does not provide for specific dimensions and/or blending techniques to achieve any improved absorbency.

Thus, an improved absorbent pad is needed which has improved biodegradability and yet maintains a high absorbency rate.

### SUMMARY OF THE INVENTION

In the present invention there is provided an absorbent pad for absorbing liquids exuded from a body, comprising a planar body having a top surface, a bottom surface and an outer perimeter defining a middle portion; a particulate blended and dispersed within at least one of the top surface, the bottom surface or the middle portion of the planar body; and, the particulate having greater absorbent characteristics than said planar body and having a maximum dimension of 1/4" (6.4mm).

Preferably, the particulate is about 1/16" (1.6mm) in dimension, the planar body comprises a non-woven fabric, particulate is peat moss and the particulate is blended with the planar body in a range of between 1 to 30% (by volume).

It is further preferable the particulate blended with the planar body is in a range of between 3 to 20% (by volume).

Desirably, the planar body is hydrophilic, further comprises a liquid impervious layer adjoined beneath the bottom surface and the particulate is embedded in at least a portion of the planar body.

It is further desirable the particulate is embedded in more than one portion of the planar body and the planar body further comprises a liquid pervious layer adjoined above the top surface, the liquid pervious layer enabling moisture exuding from the body to pass from the body to the absorbent core and preventing the moisture from returning into contact with the body exuding the moisture.

### BRIEF DESCRIPTION OF THE DRAWINGS

Figure 1 is an exploded perspective view showing an absorbent pad in an assembled form; and,

Figure 2 is a cross-sectional view illustrating the sheet of the present invention having particulates blended in different portions of the pad.

### DETAILED DESCRIPTION OF THE INVENTION

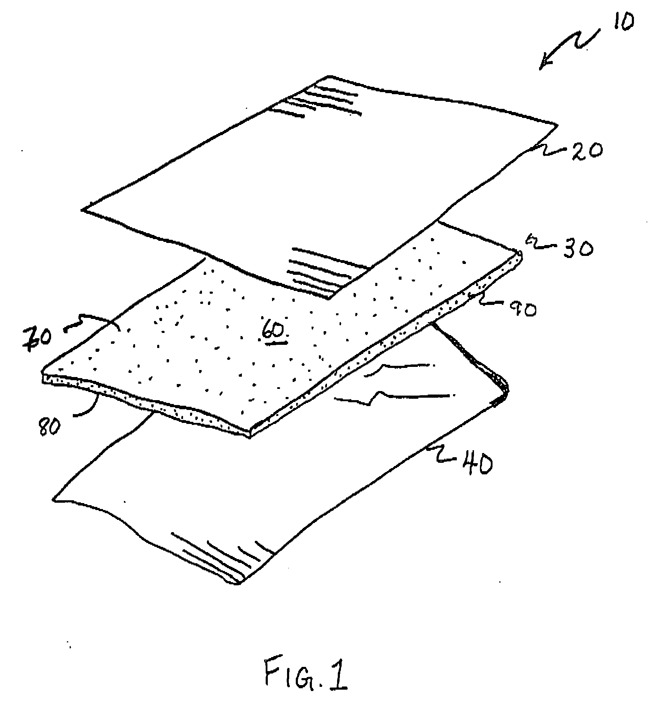

In Figure 1, there is shown an absorbent pad 10 for reducing injury attributed to moisture in direct contact with an individual's skin. The top layer 20 is generally a woven fabric such as cotton or polyester or a combination thereof. Alternatively, the top layer can be formed from a knit polyester or cotton combination.

The absorbent core 30 is generally formed from a combination of polyester and rayon and/or viscose blended together. When 100% polyester yarn is used the blend is formed and the non-woven layer needle punched. The layer can have weights varying from 3 ounces per square yard (85g per 0.836m²) up to 15 ounces per square yard (425g per 0.836m²) and include 65% polyester and 35% rayon blended with a particulate. However, as would be understood by those skilled in the art, the ratio of polyester to rayon can vary depending on the final product requirements.

The bottom layer or backing 40 is generally a strong hydrophobic material typically made from a polyethylene which maintains fluid from the absorbing core away from the bed or chair on which it is situated. The backing need only be waterproof and can be made from varying materials such as PVC, polyurethane, rubber or some other combination which effectively protects the object it is positioned on.

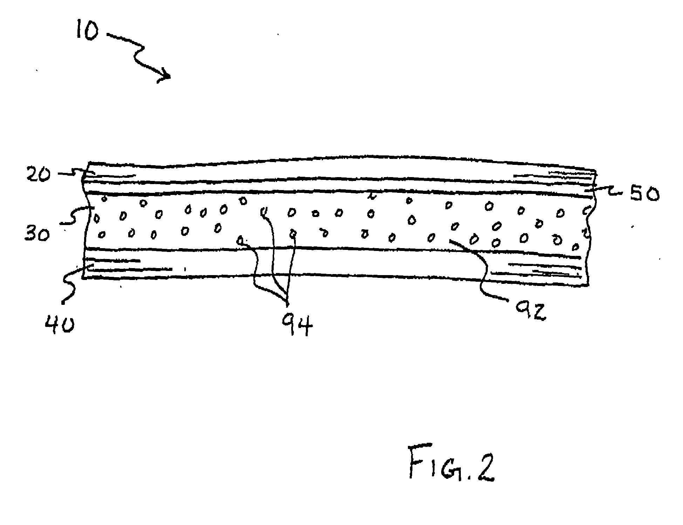

Optionally, an additional acquisition layer 50 (see Figure 2), which is often a denser cellulosic wood pulp may be provided. Generally, this additional layer provides added comfort by keeping the distance between the absorbent core and the top surface and minimizing wetback. Typically, 3 to 4 ounces per square yard (85g to 113g per 0.836m²) of non-woven loft can be used. It has also been considered that a spacer fabric in a honeycomb design can be used which provides for the desired spacing between the top surface 20 and the absorbent core 30.

The layers 20, 30, 40 and 50 are quilted together using conventional stitching techniques or the layers can optionally be combined by using conventional laminating methods to maintain the shape and integrity of the product.

In Figures 1 and 2, the absorbent core 30 is a generally planar body or sheet 60 having a top surface 70, a bottom surface 80 and an outer perimeter 90. The outer perimeter 90 defines a middle portion 92 of the sheet 60.

A particulate material 94 is blended and disbursed within at least a portion of the planar body or pad 60 such as the top surface 70, the bottom surface 80, the outer perimeter 90 or the middle portion such that the particulate is at least partially embedded in the sheet 60. The particulate material 94 is of a dimension of 1/4" (6.4mm). Preferably, the dimension is 1/16" (1.6mm) or less and the particulate material is peat moss which is sifted using a 16 mesh screen. Surprisingly, the blended absorbent core 30 having the finer particulate 94 results in an increase of 3% to 12% in absorbency based on saturation tests.

The peat moss was harvested from a peat bog which had been previously cleared, leveled and drained. During the period from May to September, on dry days the peat bog is first harrowed to fluff up and dry the top 2-3cm of peat, and this is then vacuumed into 30m³ drums on tractor drawn vacuum harvesters. The thickness of peat moss vacuumed on each pass is about 0.5cm. The peat moss is then dumped into piles at the end of the bog, stored and then trucked to the plant throughout the year into stockpiles. There the peat is dumped into a hopper by a wheeler loader, conveyed to a vibrating screener to screen out large clumps, roots and any other foreign material, dropped into the end of a propane fired drum dryer, blown up through a long drying tube to a cyclone where the dried peat moss is separated from the steam/hot air mixture, and conveyed into the plant where it is bagged into plastic bags, either compressed or loose filled. Depending on the moisture level of the harvested peat, the peat is usually passed twice through the screening/drying process before it is bagged in order to get it dry enough.

In addition to the above, a secondary process involves separators screening the peat moss to remove undesirable sizes before the peat moss is dropped onto the soaker layer of the bed sheet.

It has been determined that the finer the particulate size, the faster the absorbency of fluid. For example, the range of the particulate in the absorbent core 30 is generally between 1 to 30% (by volume) and is preferably between 3 to 20% (by volume). It has been found that larger pieces of particulate 94 provide for similar absorbency characteristics but generally also require an increase in the time required to absorb the moisture or liquid which results in prolonged exposure of the skin to the liquid.

Blending of the particulate material 94 is accomplished through means such as the use of a blow spray (not shown) which can disperse the peat moss particulate 94 on either of the top surface 20, bottom surface 40 or alternatively on both sides simultaneously such that the peat moss is embedded in at least a portion of the absorbent core. Further, it has also been contemplated that the particulate 94 can be embedded in the middle portion 92 of the absorbent core 30.

Alternatively, the blending of the peat moss particulate 94 is effected by combining the polyester and/or rayon fibers in a container (not shown) after which conventional needle punch methods are applied to the non-woven layer.

Conventional stitching methods are used to combine the top layer 20, the absorbent core 30, which includes the particulate 94 embedded in at least a portion of the layer and/or dispersed in one or more surfaces of the layer, and the bottom layer 40. Typically the final pad product is produced in widths of 36" (91.4cm), 72" (183cm) or 90" (229cm) to be packaged for shipment.

It will be understood by those skilled in the art that additional layers can be inserted in the pad product other than those set out herein above, if desirable.

The present invention provides for an improved absorbency article having improved biodegradability and increased absorbency characteristics.

## Claims

1. An absorbent pad (10) for absorbing liquid exuded from a body, comprising:
- a top layer (20) being liquid pervious to permit the liquid to flow through the layer;
- a planar body (60) beneath said top layer (20) having a top surface (70), a bottom surface (80) and an outer perimeter (90) defining a middle portion (92);
- a particulate blended and dispersed within at least one of said top surface (70), said bottom surface (80) or said middle portion (92) of said planar body (60);
- said particulate having greater absorbent characteristics than said planar body (60) and having a maximum dimension of 1/4" (6.4mm); and
- a bottom layer (40) beneath said planar body (60) impervious to liquid such that any liquid reaching the bottom layer is incapable of flow therethrough.

2. An absorbent pad (10) as claimed in Claim 1, wherein said particulate has a dimension of about 1/16" (1.6mm).

3. An absorbent pad (10) as claimed in Claim 1 or 2, wherein said planar body (60) comprises a non-woven fabric.

4. An absorbent pad (10) as claimed in Claims 1, 2 or 3, wherein said particulate is peat moss.

5. An absorbent pad (10) as claimed in any preceding Claim, wherein said particulate is blended with said planar body (60) in a range of between 1 to 30% (by volume).

6. An absorbent pad (10) as claimed in any preceding Claim, wherein said particulate is blended with said planar body (60) in a range of between 3 to 20% (by volume).

7. An absorbent pad (10) as claimed in any preceding Claim, wherein said planar body (60) is hydrophilic.

8. An absorbent pad (10) as claimed in any preceding Claim, wherein said liquid impervious bottom layer (40) is adjoined beneath said bottom surface (80).

9. An absorbent pad (10) as claimed in any preceding Claim, wherein said particulate is embedded in more than one of said top surface (70), said bottom surface (80) and said middle portion (92) of said planar body (60).

10. An absorbent pad (10) as claimed in any preceding Claim, wherein said liquid pervious top layer (20) is adjoined to said top surface (70) of said planar body (60), said liquid pervious top layer (20) enabling moisture exuding from said body to pass from said body to said planar body (60) and inhibiting said moisture from returning into contact with said body exuding said moisture.

11. A method for preparing an absorbent pad (10) as claimed in any one of claims 1 to 10, comprising the steps of:
- blending said particulate in at least one of said top surface (70), said middle portion (92) or said bottom surface (80) with a spray nozzle; and
- combining said top layer (20), said planar body (60) and said bottom layer (40) to form an absorbent pad (10).
